# EUROPEAN PATENT APPLICATION

(11) **EP 3 470 404 A1**
(43) Date of publication of application: **17.04.2019**
(21) Application number: 16904121.7
(22) Date of filing: 31.05.2016
(51) Int. Cl.: C07D 307/36, C07D 333/16, C07D 207/333, A61K 31/34, A61K 31/381, A61K 31/40

(54) **FIVE-MEMBERED HETEROCYCLIC DERIVATIVE, METHOD FOR PRODUCING SAME, AND PHARMACEUTICAL COMPOSITION COMPRISING SAME**

(71) Applicant: Industry Academic Cooperation Foundation, Hallym University, Chuncheon-si, Gangwon-do 24252 (KR); YD Life Science Co., Ltd., Seongnam-si, Gyeonggi-do 13207 (KR)
(72) Inventor: SUH, Hong-Won, Chuncheon-si Gangwon-do 24432 (KR); LIM, Soon-Sung, Chuncheon-si Gangwon-do 24278 (KR); PARK, Soo-Hyun, Chuncheon-si Gangwon-do 24267 (KR); JUNG, Sung-Jun, Suwon-si Gyeonggi-do 16295 (KR); HWANG, Seung-Hwan, Namyangju-si Gyeonggi-do 12235 (KR); LEE, Jae-Ryeong, Seoul 01233 (KR); CHOI, Jong-Gil, Seoul 06570 (KR); KIM, Jae-Yoon, Namyangju-si Gyeonggi-do 12208 (KR); KANG, Beom-Goo, Chuncheon-si Gangwon-do 24378 (KR); LEE, Han-Kyu, Bucheon-si Gyeonggi-do 14496 (KR)
(74) Representative: Adam, Holger
(86) International application number: PCT/KR2016/005763
(87) International publication number: WO 2017/209322

(57) **Abstract**

The present invention relates to a five-membered heterocyclic compound having an analgesic effect and a pharmaceutical composition comprising a derivative of the five-membered heterocyclic compound. More specifically, the present invention relates to a method for producing a five-membered heterocyclic compound having few side-effects and a broad analgesic effect, including pain relief for neuropathy, and to a pharmaceutical composition comprising the five-membered heterocyclic compound.

## Description

### Technical Field

The present invention relates to pharmaceutical compositions containing a 5-membered heterocyclic compound having an analgesic effect and a derivative thereof. More specifically, the present invention relates to a method for preparing a 5-membered heterocyclic compound having less side effects and an analgesic effect on widespread diseases including neuropathic pain, and to a pharmaceutical composition containing the same.

### Background Art

Currently, anti-inflammatory analgesic drugs, such as aspirin and Tylenol, are mainly used as analgesic drugs, and morphine-based drugs are frequently used for severe pain. As for recently developed novel pain inhibitors, "Rylomine", which is a nasal morphine sprayer developed as a spray type narcotic analgesic drug by the pain cure specialty development company, Javelin Pharmaceuticals, was confirmed to show an equal analgesic effect to intravenous morphine in the treatment of moderate to severe post-operative pain in later phase II trials, and Xinlong Biotech Co., Ltd in Shanghai, China has developed the novel drug "Achilles" that has an effect on pain caused by cancers associated with the stomach, bowels, breast, lungs, and kidneys. The new analgesic drug, "Prialt", containing a component extracted from the poison of a sea snail, was first developed by the Irish pharmaceutical company, Elan, and first marketed in the UK.

In Korea, there is a case for a joint research and licensing agreement with the Germany's multinational pharmaceutical company, Schwarz Pharma Inc., in February 2004 for developing a capsaicin antagonist developed as a non-analgesic analgesic drug.

As society ages, patients with degenerative arthritis and back pain as well as chronic malignant tumor-related pain are increasing every year. However, existing opium preparations, such as morphine, limit use to general persons due to narcotic action thereof, and moreover, pain itself is considered as a disease, and the interest of pain is increasing than ever. Therefore, the demand for pain relievers is expected to significantly increase in the future, and the development of analgesic drugs for pain that does not respond to existing analgesic drugs is greatly required.

In addition, there have been many reports of pain patients who do not respond to existing narcotic and non-narcotic analgesic drugs, and existing opium-like preparations, which are known to have a relatively high analgesic effect, limit use thereof due to narcotic characteristics and serious side effects thereof. Therefore, the present inventors intended to develop natural materials with new analgesic efficacy without narcotic characteristics and side effects.

Acetaminophen and aspirin are currently used as analgesic drugs, but these analgesic drugs need to be administered to adults in a high dose of about 1-4 g per day, which causes side effects, such as gastrointestinal disorders, allergies, and hepatotoxicity. Therefore, the development of new analgesic drugs having an excellent analgesic effect even at a low dose and having no side effects is urgently required.

### Detailed Description of the Invention

### Technical Problem

An aspect of the present invention is to provide a compound of chemical formula 1 below or a pharmaceutically acceptable salt thereof: 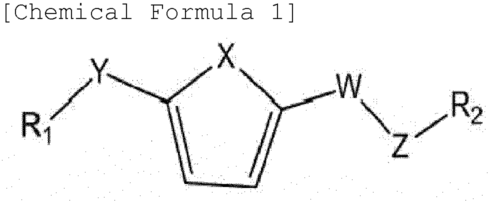 wherein,
X is O, S, or NH;
Y is selected from the group consisting of -C(O)-, -C(S)-, -C(NH)-, and unsubstituted or substituted linear or branched C₁-C₆ alkylene;
R₁ and R₂ each are independently selected from the group consisting of H, -NR'R", -C(O)NR'R", -OR₃, halogen, unsubstituted or substituted linear or branched C₁-C₁₀ alkyl, unsubstituted or substituted C₂-C₁₀ alkenyl, unsubstituted or substituted C₃-C₂₀ cycloalkyl, unsubstituted or substituted C₆-C₂₀ aryl, unsubstituted or substituted C₃-C₂₀ heterocyclyl, unsubstituted or substituted C₅-C₁₂ heteroaryl, and C₁-C₆ alkyl C₅-C₁₂ heteroaryl;
R₃ is H or linear or branched C₁-C₆ alkyl;
W is selected from the group consisting of a bond, -C(O)-NH-, -NH-C(O)-, and -C₁-C₆ alkylene-NR'R";
Z is a bond or linear or branched C₁-C₁₀ alkylene; and
R' and R" each are independently selected from the group consisting of H, unsubstituted or substituted linear or branched C₁-C₆ alkyl; C₃-C₁₂ cycloalkyl; unsubstituted or substituted 5- to 12-membered heterocyclyl; C₂-C₆ alkenyl; C₆-C₁₂ aryl; C₁-C₆ alkyl C₆-C₁₂ aryl; unsubstituted or substituted 5- to 12-membered heteroaryl; C₁-C₆ alkyl C₅-C₁₂ heteroaryl; C₁-C₆ alkyl sulfonyl; C₆-C₁₂ aryl sulfonyl; C₆-C₂₀ aryl sulfonamide; mercapto; hydroxy (C₁-C₁₀) alkyl; -OH; C₁-C₆ alkoxy; C₁-C₆ alkoxy C₁-C₁₀ alkyl; hydroxy C₁-C₁₀ alkyl; C₁-C₆ haloalkoxy; halogen; -COOH; -CHO; -CN; -C₁-C₆ alkyl carbonyl; -C₁-C₆ alkyl oxy carbonyl; and mono- or di-C₁-C₆ alkyl carbamoyl,
wherein R' and R", together with N, may form a saturated or unsaturated 3- to 6-membered ring, and the ring may be further substituted with C₆-C₂₀ aryl or C₅-C₁₂ heteroaryl or may form a fused ring together therewith, and
wherein the substituted alkyl, substituted alkenyl, substituted cycloalkyl, substituted aryl, substituted heterocyclyl, and substituted heteroaryl mean that the alkyl, alkenyl, cycloalkyl, aryl, heterocyclyl, and heteroaryl each are independently substituted with at least one substituent selected from the group consisting of C₁-C₁₀ alkyl; C₃-C₁₂ cycloalkyl; C₂-C₁₀ alkenyl; C₆-C₂₀ aryl; (C₁-C₆) alkyl (C₆-C₁₂) aryl; C₅-C₁₂ heteroaryl; C₁-C₆ alkyl sulfonyl; C₆-C₁₂ aryl sulfonyl; C₆-C₂₀ aryl sulfonamide; C₁-C₆ alkyl thio; mercapto; hydroxy(C₁-C₁₀)alkyl; -OH; C₁-C₆ alkoxy; C₆-C₂₀ aryloxy; (C₁-C₆)alkoxy(C₁-C₁₀)alkyl; C₁-C₆ haloalkoxy; -NO₂; halogen; - COOH; -CHO; -CN; -C₁-C₆ alkyl carbonyl; -C₁-C₆ alkyl oxy carbonyl; -CONR'R"; -NR'R"; N-hydroxyl carbamoyl; and mono- or di-C₁-C₁₀ alkyl carbamoyl.

Another aspect of the present invention is to provide a pharmaceutical composition for treating or relieving neuropathic pain, acute pain, chronic pain, abdominal pain, toothache, menstrual pain, backache, frozen shoulder pain, herpes zoster, trigeminal neuralgia, cancer pain, diabetic neuropathy, post-operative pain, migraine, or joint pain, the pharmaceutical composition containing a compound of chemical formula 1, or a hydrate or pharmaceutically acceptable salt thereof.

Still another aspect of the present invention is to provide a method for treating a symptom or relieving pain in a patient with severe pain. The method includes administrating, to the patient, a therapeutically effective amount of a compound of chemical formula 1, or a hydrate or pharmaceutically acceptable salt thereof.

Still another aspect of the present invention is to provide an anesthetic use of a compound of chemical formula 1, or a hydrate or pharmaceutically acceptable salt thereof.

Still another aspect of the present invention is to provide a method for preparing a compound of chemical formula 1, or a hydrate or pharmaceutically acceptable salt thereof.

### Technical Solution

Hereinafter, the present invention will be described in detail with reference to specific embodiments thereof, examples of which are illustrated in the accompanying structural formulas and formulas. The invention will be described in conjunction with the listed embodiments, which include all alternatives, modifications, and equivalents that may be included within the scope of the invention as defined in claims. A person skilled in the art would recognize reagents and the like used in the methods and synthesis similar or equivalent to those described herein, which can be used in the implementation of the present invention.

### Definitions

As used herein, the term "alkyl" refers to a saturated, linear or branched monovalent hydrocarbon, and here the alkyl radical may be independently optionally substituted with at least one substituent described herein. In an embodiment, the alkyl radical has 1-18 carbon atoms (C₁-C₁₈). In another embodiment, the alkyl radical may have C₁-C₁₂, C₁-C₁₀, C₁-C₈, C₁-C₆, C₁-C₅, C₁-C₄, or C₁-C₃ carbon atoms. An example of the alkyl group may include methyl, ethyl, 1-propyl, 2-propyl, 1-butyl, 2-methyl-1-propyl, 2-butyl, 2-methyl-2-propyl, 1-penyl, 2-pentyl(-CH(CH₃)CH₂CH₂CH₃), 3-pentyl(-CH(CH₂CH₃)₂), 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, and 1-octyl.

As used herein, the term "alkenyl" refers to a linear or branched monovalent hydrocarbon radical including at least one unsaturated site, that is, a carbon-carbon double bond, and here the alkenyl radical may be independently optionally substituted with at least one substituent defined herein, and includes radicals having "cis" and "trans" orientations, or alternatively, "E" and "Z" orientations. In one embodiment, the alkenyl radical has 2-18 carbon atoms (C₂-C₁₈). In another embodiment, the alkenyl radical may have C₂-C₁₂, C₂-C₁₀, C₂-C₈, C₂-C₆, or C₂-C₃ carbon atoms. Non-limiting examples thereof may include ethenyl or vinyl, prop-1-enyl, prop-2-enyl, 2-methylprop-1-enyl, but-1-enyl, but-2-enyl, but-3-enyl, buta-1,3-dienyl, 2-methylbuta-1,3-diene, hex-1-enyl, hex-2-enyl, hex-3-enyl, hex-4-enyl, and hexa-1,3-dienyl.

As used herein, the term "cycloalkyl" refers to a non-aromatic, saturated or partially unsaturated hydrocarbon ring group, and the cycloalkyl groups may be independently optionally substituted with at least one substituent described herein. In one embodiment, the cycloalkyl group has 3-20 carbon atoms (C₃-C₂₀). In another embodiment, the cycloalkyl may have C₃-C₈, C₃-C₁₀, C₅-C₁₀, or C₃-C₂₀ carbon atoms. In another embodiment, the cycloalkyl group may have C₃-C4, C₃-C₆, or C₅-C₆ carbon atoms as a monocycle. In still another embodiment, the cycloalkyl group may have C₇-C₁₂ carbon atoms as a bicycle. Examples of the monocyclic cycloalkyl include cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, cyclononyl, cyclodecyl, cycloundecyl, and cyclododecyl. The bicyclic cycloalkyl having 7 to 12 ring atoms may include, but are not limited to, [4,4], [4,5], [5,5], [5,6], or [6,6] ring systems Exemplary cross-linked bicyclic cycloalkyl include, but are not limited to, bicyclo[2.2.1] heptane, bicyclo[2.2.2] octane, and bicyclo[3.2.2] nonane.

As used herein, the term "aryl" refers to a cyclic aromatic hydrocarbon group that is independently optionally substituted with at least one substituent disclosed herein. In one embodiment, the aryl group has 6-20 carbon atoms (C₆-C₂₀). In another embodiment, the aryl group may have C₆-C₉ carbon atoms. In still another embodiment, the aryl group is a C₆ aryl group. The aryl may include a bicyclic group including an aromatic ring including a fused non-aromatic or partially saturated ring. Exemplary aryl groups include, but are not limited to, phenyl, naphthalenyl, anthracenyl, indenyl, indanyl, 1,2-dihydronaphthalenyl, and 1,2,3,4-tetrahydronaphthyl. In an embodiment, the aryl includes phenyl. The substituted phenyl or substituted aryl means a phenyl group or aryl group that is substituted with 1, 2, 3, 4, or 5, for example, 1-2, 1-3, or 1-4 substituents selected from the group specified herein. In an embodiment, an optional substituent on aryl is selected from halogen (F, Cl, Br, I), hydroxy, protected hydroxy, cyano, nitro, alkyl (for example, C₁-C₆ alkyl), alkoxy (for example, C₁-C₆ alkoxy), benzyloxy, carboxy, protected carboxy, carboxymethyl, protected carboxymethyl, hydroxymethyl, protected hydroxymethyl, aminomethyl, protected aminomethyl, trifluoromethyl, alkylsulfonylamino, alkylsulfonylaminoalkyl, arylsulfonylamino, arylsulfonylaminoalkyl, heterocyclylsulfonylamino, heterocyclylsulfonylaminoalkyl, heterocyclyl, aryl, or other groups specified. One or more methyne (CH) and/or methylene (CH₂) groups in these substituents may in turn be substituted with a similar group as those denoted above. The fused aryl ring may also be substituted with any, e.g., 1, 2, or 3 substituents specified herein, in the same manner in which the alkyl group is substituted.

As used herein, the terms "heterocycle," "heterocyclyl", and "heterocyclic ring" are used interchangeably herein, and refer to (i) a saturated or a partially unsaturated cyclic group (i.e., having one or more double and/or triple bonds within the ring) ("heterocycloalkyl"), or (ii) an aromatic cyclic group ("heteroaryl"), and at each case, at least one ring atom is a heteroatom independently selected from nitrogen, oxygen, phosphorus, and sulfur, and the other ring atom(s) are C. The heterocyclyl group may be optionally substituted with at least one substituent disclosed herein. In an embodiment, the heterocyclyl includes a monocycle or bicycle having one to nine carbon ring members (C₁-C₉), and the other ring atom is a heteroatom selected from N, O, S, and P. In another embodiment, the heterocyclyl includes a monocycle or bicycle having C₁-C₅, C₃-C₅ and C₄-C₅ atoms, and the other ring atom is a heteroatom selected from N, O, S, and P. In a particular embodiment, the heterocyclyl includes a 3- to 12-membered ring, a 3- to 10-membered ring, a 3- to 7-membered ring, or a 3- to 6-membered ring containing at least one heteroatom independently selected from N, O, S, and P. In another embodiment, the heterocyclyl includes a monocyclic 3-, 4-, 5-, 6-, or 7-membered ring containing at least one heteroatom independently selected from N, O, S, and P. In still another embodiment, the heterocyclyl includes a bi- or polycyclic, spiro or bridged 4-, 5-, 6-, 7-, 8-, 9-, 10-, 11-, or 12-membered ring system containing at least one heteroatom independently selected from N, O, S, and P. Non-limiting examples of the bicycle system include a [3,5], [4,5], [5,5], [3,6], [4,6], [5,6], or [6,6] system. Non-limiting examples of the bridged ring system include [2.2.1], [2.2.2], [3.2.2], and [4.1.0] assignments and have one to three heteroatoms selected from N, O, S, and P. In still another embodiment, the heterocyclyl includes a spiro group having one to four heteroatoms selected from N, O, S, and P. The heterocyclyl group may be a carbon-linked or heteroatom-linked group. The "heterocyclyl" includes a heterocyclyl group fused to a cycloalkyl group and a heterocyclyl group fused to an aryl group.

The term "heteroaryl" refers to an aromatic carbocyclic radical, and here one or more ring atoms are a hetero atom independently selected from nitrogen, oxygen, and sulfur, and the other ring atom(s) are carbon. The heteroaryl group may be optionally substituted with at least one substituent disclosed herein. In an embodiment, the heteroaryl group contains 1-9 carbon ring atoms (C₁-C₉). In another embodiment, the heteroaryl group has C₁-C₅, C₃-C₅, or C₄-C₅ carbon atoms. In an embodiment, exemplary heteroaryl groups include a 5- to 12-membered ring, a 5- to 10-membered ring, or a 5- to 6-membered ring containing at least one heteroatom independently selected from nitrogen, oxygen, and sulfur, or a monocyclic aromatic 5-, 6-, and 7-membered ring. In still another embodiment, exemplary heteroaryl groups include a ring system of 10 or less carbon atoms, and in still another embodiment, include a ring system of 9 carbon atoms, and here at least one aromatic ring contains at least one hetero atom independently selected from nitrogen, oxygen, and sulfur. The "heteroaryl" includes a heteroaryl group fused to an aryl, cycloalkyl, or another heterocyclyl group. Non-limiting examples of the heteroaryl group include pyridinyl, imidazolyl, imidazopyridinyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, furazanyl, benzofurazanyl, benzothiophenyl, benzothiazolyl, benzoxazolyl, quinazolinyl, quinoxalinyl, naphthyridinyl, pyrrolopyridinyl, pirazolopyrimidinyl, and furopyridinyl.

The "pharmaceutically acceptable salt," as used herein, refers to a pharmaceutically acceptable organic or inorganic salt of a compound of chemical formula 1. Exemplary salts include, but are not limited to, sulfate, citrate, acetate, oxalate, chloride, bromide, iodide, nitrate, bisulfate, phosphate, acid phosphate, isonicotinate, lactate, salicylate, citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)). The pharmaceutically acceptable salt may involve the inclusion of other molecules, such as an acetate ion, succinate ion, or another counter ion. The counter ion may be any organic or inorganic residue that stabilizes the charges on a parent compound. Furthermore, the pharmaceutically acceptable salt may have an atom exhibiting at least one charge in a structure thereof. In cases where an atom exhibiting multiple charges is a part of the pharmaceutically acceptable salt, the pharmaceutically acceptable salt may have a multiple counter ion. Therefore, the pharmaceutically acceptable salt may have at least one charged atom or at least one counter ion.

The term "compound of the present invention", unless otherwise stated, includes a compound of chemical formula 1, or a stereoisomer, tautomer, solvate, prodrug, and salt thereof (such as a pharmaceutically acceptable salt). Unless otherwise stated, the structures depicted herein are also meant to include different compounds only in the presence of one or more isotopically enriched atoms. For example, compounds of chemical formula 1 in which at least one hydrogen atom is replaced by deuterium or tritium or at least one carbon atom is replaced by a carbon atom of 13C- or 14C-within the scope of the present invention.

In an embodiment, there are provided a compound of chemical formula 1 or a hydrate or a pharmaceutically acceptable salt thereof, and a pharmaceutical composition, which are useful for treating or alleviating pain in a patient suffering from chronic malignancies, degenerative arthritis, back pain-related diseases, and the like.

An aspect of the present invention includes a compound of chemical formula 1 below or a pharmaceutically acceptable salt thereof: 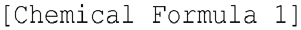 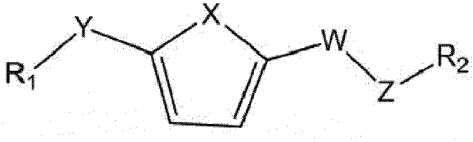 wherein,
X is O, S, or NH;
Y is selected from the group consisting of -C(O)-, -C(S)-, -C(NH)-, and unsubstituted or substituted linear or branched C₁-C₆ alkylene;
R₁ and R₂ each are independently selected from the group consisting of H, -NR'R", -C(O)NR'R", -OR₃, halogen, unsubstituted or substituted linear or branched C₁-C₁₀ alkyl, unsubstituted or substituted C₂-C₁₀ alkenyl, unsubstituted or substituted C₃-C₂₀ cycloalkyl, unsubstituted or substituted C₆-C₂₀ aryl, unsubstituted or substituted C₃-C₂₀ heterocyclyl, unsubstituted or substituted C₅-C₁₂ heteroaryl, and C₁-C₆ alkyl C₅-C₁₂ heteroaryl;
R₃ is H or linear or branched C₁-C₆ alkyl;
W is selected from the group consisting of a bond, -C(O)-NH-, -NH-C(O)-, and -C₁-C₆ alkylene-NR'R";
Z is a bond or linear or branched C₁-C₁₀ alkylene; and
R' and R" each are independently selected from the group consisting of H, unsubstituted or substituted linear or branched C₁-C₆ alkyl; C₃-C₁₂ cycloalkyl; unsubstituted or substituted 5- to 12-membered heterocyclyl; C₂-C₆ alkenyl; C₆-C₁₂ aryl; C₁-C₆ alkyl C₆-C₁₂ aryl; unsubstituted or substituted 5- to 12-membered heteroaryl; C₁-C₆ alkyl C₅-C₁₂ heteroaryl; C₁-C₆ alkyl sulfonyl; C₆-C₁₂ aryl sulfonyl; C₆-C₂₀ aryl sulfonamide; mercapto; hydroxy (C₁-C₁₀) alkyl; -OH; C₁-C₆ alkoxy; C₁-C₆ alkoxy C₁-C₁₀ alkyl; hydroxy C₁-C₁₀ alkyl; C₁-C₆ haloalkoxy; halogen; -COOH; -CHO; -CN; -C₁-C₆ alkyl carbonyl; -C₁-C₆ alkyl oxy carbonyl; and mono- or di-C₁-C₆ alkyl carbamoyl,
wherein R' and R", together with N, may form a saturated or unsaturated 3- to 6-membered ring, and the ring may be further substituted with C₆-C₂₀ aryl or C₅-C₁₂ heteroaryl or may form a fused ring together therewith.

In one embodiment of the present invention, X is O.

In one embodiment of the present invention, Y is - C(O)-.

In an embodiment of the present invention, X is O; Y is -C(O)-; R₁ is unsubstituted or substituted C₆-C₂₀ aryl, unsubstituted or substituted C₅-C₁₂ heteroaryl, or -NR'R", wherein R' and R" are as defined in chemical formula 1 above.

In an embodiment of the present invention, W is - C(O)-NH- or linear or branched C₁-C₁₀ alkylene; Z is a bond or linear or branched C₁-C₁₀ alkylene; R₂ is -NR'R", -C(O)NR'R", -C₁-C₆ alkyloxy carbonyl, -COOH, C₆-C₂₀ aryl, unsubstituted or substituted C₃-C₂₀ heterocyclyl, or unsubstituted or substituted C₅-C₁₂ heteroaryl, wherein R' and R" are as defined in chemical formula 1 above.

In an embodiment of the present invention, W is - C(O)-NH-; Z is linear or branched C₁-C₁₀ alkylene; R₂ is - NR'R", -C(O)NR'R", -C₁-C₆ alkyloxy carbonyl, -COOH, C₆-C₂₀ aryl, unsubstituted or substituted C₃-C₂₀ heterocyclyl, or unsubstituted or substituted C₅-C₁₂ heteroaryl, wherein R' and R" are as defined in chemical formula 1 above.

In a preferred embodiment of the present invention, the substituted alkyl, substituted alkenyl, substituted cycloalkyl, substituted aryl, substituted heterocyclyl, and substituted heteroaryl mean that the alkyl, alkenyl, cycloalkyl, aryl, heterocyclyl, and heteroaryl each are independently substituted with at least one substituent selected from the group consisting of C₁-C₁₀ alkyl; C₃-C₁₂ cycloalkyl; C₂-C₁₀ alkenyl; C₆-C₂₀ aryl; (C₁-C₆)alkyl (C₆-C₁₂) aryl; C₅-C₁₂ heteroaryl; C₁-C₆ alkyl sulfonyl; C₆-C₁₂ aryl sulfonyl; C₆-C₂₀ aryl sulfonamide; C₁-C₆ alkyl thio; mercapto; hydroxy(C₁-C₁₀)alkyl; -OH; C₁-C₆ alkoxy; C₆-C₂₀ aryloxy; (C₁-C₆)alkoxy(C₁-C₁₀)alkyl; C₁-C₆ haloalkoxy; - NO₂; halogen; -COOH; -CHO; -CN; -C₁-C₆ alkyl carbonyl; - C₁-C₆ alkyl oxy carbonyl; -CONR'R"; -NR'R"; N-hydroxyl carbamoyl; and mono- or di-C₁-C₁₀ alkyl carbamoyl.

The compound of chemical formula 1 of the present invention contains a bi-chiral or chiral center, and thus may be present as a different stereoisomer form. All stereoisomers of the compounds of chemical formula 1, including diastereomers, enantiomers, as well as mixtures thereof, such as racemic mixtures, are intended to form a part of the present invention. In addition, the present invention includes all geometrical isomers and position isomers. For example, in cases where the compound of chemical formula 1 includes a double bond or a fused ring, both cis- and trans-forms as well as mixtures thereof are included within the range of the present invention.

The compound of the present invention is present in an unsolvated form as well as a solvated form in a pharmaceutically acceptable solvent, such as water, ethanol, or the like, and the present invention is intended to include both solvated and unsolvated forms.

Still another embodiment of the present invention includes a pharmaceutical composition containing a compound of chemical formula I or a hydrate or pharmaceutically acceptable salt thereof, and a pharmaceutically acceptable carrier, an adjuvant, or a vehicle.

Still another aspect of the present inveniton includes a pharmaceutical composition for treating or relieving neuropathic pain, acute pain, chronic pain, abdominal pain, toothache, menstrual pain, backache, frozen shoulder pain, herpes zoster, trigeminal neuralgia, cancer pain, diabetic neuropathy, post-operative pain, migraine, or joint pain, the pharmaceutical composition containing the compound of chemical formula 1 or a hydrate or pharmaceutically acceptable salt thereof.

Still another aspect of the present invention includes a method for treating a symptom or relieving pain in a patient with severe pain. The method includes administrating, to the patient, a therapeutically effective amount of a compound of chemical formula 1, or a hydrate or pharmaceutically acceptable salt thereof.

Still another embodiment of the present invention includes an anesthetic use of a compound of chemical formula 1, or a hydrate or pharmaceutically acceptable salt thereof.

The pharmaceutical composition of the present invention can be formulated into various oral or parenteral administration forms. Exemplary formations for oral administration are a tablet, a pill, a hard soft capsule, a liquid, a suspension, an emulsion, a syrup, granules, and the like, and these formulations contain, in addition to an active ingredient, a diluent (such as lactose, dextrose, sucrose, mannitol, sorbitol, cellulose, and/or glycine) or a lubricant (such as silica, talc, stearic acid, and magnesium or calcium salts thereof, and/or polyethyleneglycol). The tablet may also contain a binder, such as magnesium aluminum silicate, starch paste, gelatin, tragacanth, methylcellulose, sodium carboxymethylcellulose, and/or polyvinylpyrrolidone, and, in some cases, may contain a disintegrant, such as starch, agar, or alginic acid or a sodium salt thereof, or a boiling mixture and/or an absorbent, a colorant, a flavor, and a sweetener. The formulations may be prepared by ordinary mixing, granulation, or coating. In addition, a representative formulation for parenteral administration is a formulation for injection, which is preferably an isotonic aqueous solution or a suspension. The composition is sterilized and/or may contain an adjuvant, such as a preservative, a stabilizer, a wettable powder, or an emulsification accelerator, a salt for controlling osmotic pressure, and/or a buffer and other therapeutically beneficial substances, and may be formulated according to a conventional method.

The compound of chemical formula 1 of the present invention may be administered via any route suitable for a symptom to be treated. The suitable route includes an oral, parenteral (e.g., subcutaneous, intramuscular, intravenous, intraarterial, intradermal, intrathecal, and epidural), transdermal, rectal, nasal, topical (e.g., buccal and sublingual), vaginal, intraperitoneal, intrapulmonary and intranasal administrations. In the treatment of topical pain, the compound may be administered by intralesional administration, for example, may be administered by contact of an inhibitor and a graft prior to perfusion or transplantation. It can be confirmed that preferable routes may vary depending on the symptom of the beneficiary.

In an embodiment, a pharmaceutical effective amount of the compound of the present invention, which is administered parenterally per dose, may be in the range of about 0.01-100 mg/kg, alternatively about 0.1-20 mg/kg of body weight of the patient per day, and a typical initial range of the compound used is 0.3-15 mg/kg/day.

In still another embodiment, preferably about 5-100 mg of the compound of the present invention is contained in an oral unit dosage form, such as a tablet and a capsule. The dose to be administered to a patient may range from about 5-1000 mg of the compound of chemical formula 1. The general dose may be about 5-300 mg of the compound of chemical formula 1. The dose may be administered once daily (QID), twice daily (BID), or more often, depending on pharmacokinetic and pharmacodynamic properties, including absorption, distribution, metabolism, and release of the particular compound. In addition, toxic factors can affect the dose and administration regimen. When administered orally, a pill, a capsule, or a tablet may be administered daily or less frequently for a certain period of time. The administration regimen may be repeated with various therapy cycles.

The dosage level for a particular patient may vary depending on the patient's body weight, age, sex, health condition, and diet, the time of administration, the method of administration and excretion, and the drug combination and severity of disease.

### Advantageous Effects

The present invention provides a pharmaceutical composition containing a 5-membered heterocyclic derivative having less side effects and an excellent analgesic effect as a non-narcotic analgesic drug.

Furthermore, the present invention provides a method for preparing a 5-membered heterocyclic derivative having an excellent analgesic effect.

### Brief Description of the Drawings

FIGS. 1 to 5 show test results (writhing test) in the oral administration of 5-membered heterocyclic derivatives of the present invention.
FIGS. 6 to 9 show test results (formalin test) in the oral administration of 5-membered heterocyclic derivatives of the present invention.
FIGS. 10 to 15 are graphs showing analgesic effects of 5-membered heterocyclic derivatives for different concentrations and times in neuropathic animal models induced by vincristine.
FIG. 16 shows pKa measurement results of 5-benzoyl -N-(4-(tert-butyl amino)-3-oxopropyl)furan-2-carboxamide.
FIG. 17 shows solubility measurement results of 5-benzoyl -N-(4-(tert-butyl amino)-3-oxopropyl)furan-2-carboxamide.
FIG. 18 is a graph showing an effect of 5-benzoyl - N-(4-(tert-butyl amino)-3-oxopropyl)furan-2-carboxamide on TTX-resistant Na channels.
FIG, 19 is a graph obtained by orally administering each compound at a concentration of 40 or 80 mg/kg, and after 30, 60, and 120 minutes, the degree of analgesia was measured by a tail-flick test, in neuropathic animal models in which neuropathy was induced by abdominal administration of vincristine at 0.1 mg/kg once a day for 7 days.
FIG, 20 is a graph obtained by orally administering each compound at a concentration of 40 or 80 mg/kg, and after 30, 60, and 120 minutes therefrom, the degree of analgesia was measured by a von-Frey test, in neuropathic animal models in which neuropathy was induced by abdominal administration of vincristine at 0.1 mg/kg once a day for 7 days.

### Mode for Carrying Out the Invention

Hereinafter, the present invention will be described in detail with reference to the following examples. However, the following examples are merely for illustrating the present invention and are not intended to limit the scope of the present invention.

### Synthesis of 5-membered heterocyclic derivatives

### Example 1: Synthesis of 5-membered heterocyclic derivative (FY-05-01-I-1)

### Step 1:

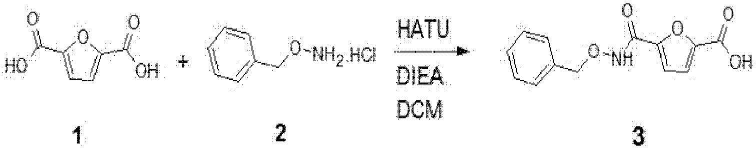

Furan-2,5-dicarboxylic acid (compound 1) (8 g, 0.05 mol, 1.0 eq), oxy benzyl amine hydrochloride (compound 2) (8 g, 0.05 mol, 1.0 eq), and HATU (25.46 g, 0.067 mol, 1.3 eq) were dissolved in DCM (100 mL), and DIEA was slowly added thereto under N₂, followed by stirring at RT for 2 hours. The reaction mixture was cooled with water. The aqueous solution was extracted with DCM (100 mL x 2) several times. The collected organic layer was washed with water, dried over anhydrous Na₂SO₄, and concentrated. The residue was purified by flash chromatography charged with silica gel (MeOH/DCM = 1/100) to give compound 3 as white solid (11.4 g, 85.6%) .

### Step 2:

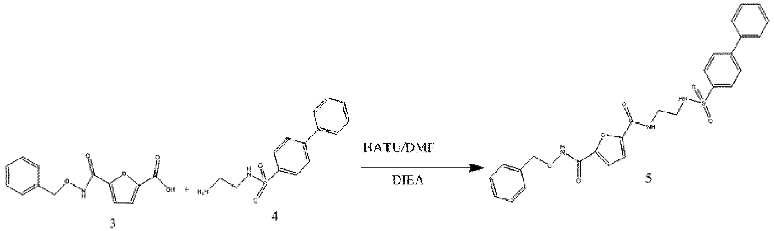

DIEA (0.6 mL) was slowly added, under N₂, to a solution obtained by dissolving HATU (450 mg, 1.15 mmol, 1.0 eq), compound 4 (275 mg, 1.37 mmol, 1.2 eq), and compound 3 (300 mg, 1.15 mmol, 1.0 eq) in DMF (10 mL), followed by stirring at RT overnight. The reaction mixture was concentrated, and the brown oil reside was diluted with water. The aqueous solution was extracted with EA (50 mL x 2) several times. The collected organic layer was washed with water, dried over anhydrous Na₂SO₄, and concentrated. The residue was purified by flash chromatography charged with silica gel (PE/EA = 1/10) to give compound 5 as pink solid (400 mg, 78%).

### Step 3:

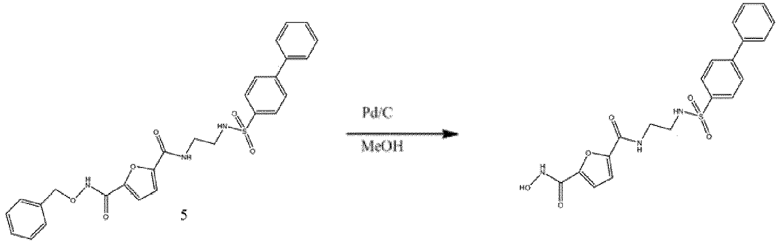

Pd/C was added to a solution obtained by dissolving compound 5 in MeOH, followed by stirring at 50°C under H₂ overnight. The reaction mixture was filtered and concentrated, and the unpurified residue was obtained. The residue was purified by preparative high performance liquid chromatography (prep-HPLC) to give a target compound as white solid (36 mg, 11%).

### Example 2: Preparation of N²-(2-(1H-indol-3-yl)ethyl)-N⁵-hydroxyfuran-2,5-dicarboxamide (FY-07-05-I-1B)

A target compound was obtained by the same method as in example 1 except that, in step 2 in example 1, compound 6 (500 mg, 3.1 mmol, 1.0 eq) below, instead of adding compound 4, was added, followed by reaction.

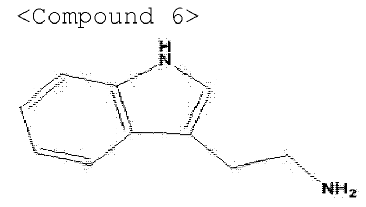

### Example 3: N²-hydroxy-N⁵-(2-(2-methyl-1H-indol-3-yl)ethyl)furan-2,5-dicarboxamide (FY-07-03-I-1)

A target compound was obtained by the same method as in example 1 except that, in step 2 in example 1, compound 7 below, instead of adding compound 4, was added, followed by reaction.

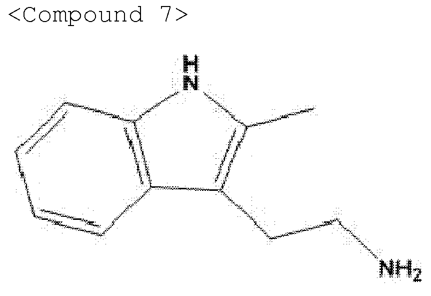

### Example 4: N²-(3-(3,4-dihydroisoquinolin-2(1H)-yl)-3-oxopropyl)-N⁵-hydroxyfuran-2,5-dicarboxamide (FY-11-03-I-1)

### Step 1:

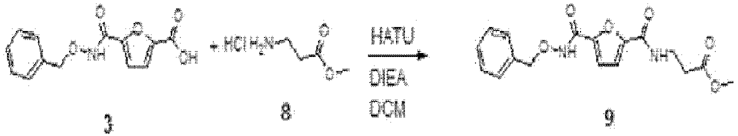

5-[N-(phenylmethoxy)carbamoyl]furan-2-carboxylic acid (compound 3) (7.5 g, 28 mmol, 1.0 eq), methyl 3-aminopropanoate hydrochloride (compound 8) (4.0 g, 28 mmol, 1.0 eq), and HATU (13.8 g, 36.4 mmol, 1.3 eq) were dissolved in DCM(100 mL), and DIEA (7.2 g, 56 mmol, 2.0 eq) was slowly added under N₂, followed by stirring at RT overnight. The reaction mixture was cooled with water. The aqueous solution was extracted with DCM (100 mL x 2) several times. The collected organic layer was washed with water, dried over anhydrous Na₂SO₄, and concentrated. The residue was purified by flash chromatography charged with silica gel (MeOH/DCM = 1/50) to give compound 9 as colorless oil (11.4 g, 85.6%).

### Step 2:

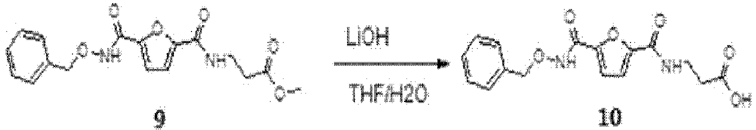

LiOH·H₂O (0.47 g, 12 mmol, 2.0 eq) was added to a solution obtained by dissolving compound 9 (2 g, 5.8 mmol, 1.0 eq) in THF/H₂O (1:1, 20 mL) cooled to 0°C followed by stirring at RT overnight. The reaction mixture was concentrated, and the residue was cooled, followed by acidification with 1N HCl of pH < 6 and extraction with DCM. Product 10 as yellow oil (1.1g, 57%) was obtained.

### Step 3:

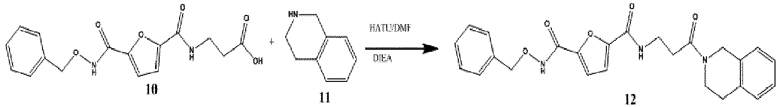

DIEA (0.6 mL) was slowly added, under N₂, to a solution obtained by dissolving HATU (570 mg, 1.5 mmol, 1.55 eq), compound 11, and compound 10 (300 mg, 0.9 mmol, 1.0 eq) in DMF (10 mL), followed by stirring at RT overnight. The reaction mixture was concentrated, and the brown oil reside was diluted with water. The aqueous solution was extracted with EA(50 mL x 2) several times. The collected organic layer was washed with water, dried over anhydrous Na₂SO₄, and concentrated. The residue was purified by flash chromatography charged with silica gel (PE/EA = 1/10) to give compound 12 as gray solid (190 mg, 45%).

### Step 4:

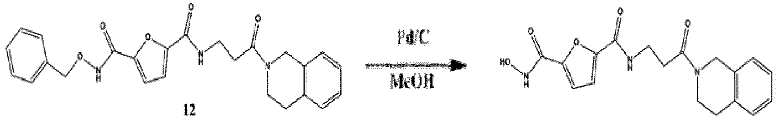

Pd/C was added to a solution obtained by dissolving compound 7 (140 mg, 0.3 mmol, 1.0 eq) in MeOH (20 mL), and the reaction product was stirred under H₂ at 50°C overnight. The reaction mixture was filtered and concentrated, and the unpurified residue was obtained. The residue was purified by preparative high performance liquid chromatography (prep-HPLC) to give target compound as gray solid (8 mg, 7%).

### Example 5: N²-(benzyloxy)-N⁵-(3-oxo-3-(quinolin-6-ylamino)propyl)furan-2,5-dicarboxamide (FY-10-01-I-2)

A target compound was obtained by using the same method as in example 4 except that in step 1 in example 4, methyl 3-aminobutanoate hydrochloride, instead of methyl 3-aminopropanoate hydrochloride, was added, and in step 3, compound 13 below, instead of compound 11, was added, followed by reaction.

### <Methyl 3-aminobutanoate hydrochloride>

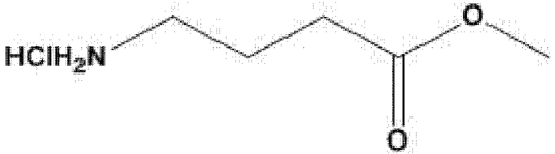

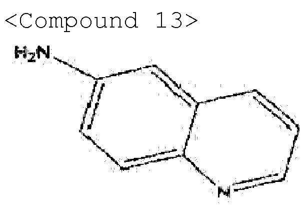

### Example 6: 5-Benzoyl-N-(4-(tert-butylamino)-3-oxopropyl)furan-2-carboxamide (FY-05-04-I-06)

### Step 1:

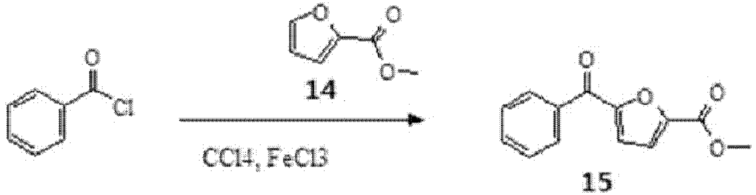

Compound 14 (4.438 g, 37.2 mmol, 1.2 eq) was slowly added, under N₂, to a solution obtained by dissolving FeCl (50 mg, 3.1 mmol, 1% eq) and benzoyl chloride (4 g, 31 mmol, 1.0 eq) in CCl₄ (160 mL), followed by stirring under flux for 2 days. The reaction mixture was cooled with water. The aqueous solution was extracted with DCM (50 mL x 2) several times. The collected organic layer was washed with water, dried over anhydrous Na₂SO₄, and concentrated. The residue was purified by chromatography charged with silica gel (PE/EA = 20/1) to give compound 15 as yellow solid (2.9 g, 40%).

### Step 2:

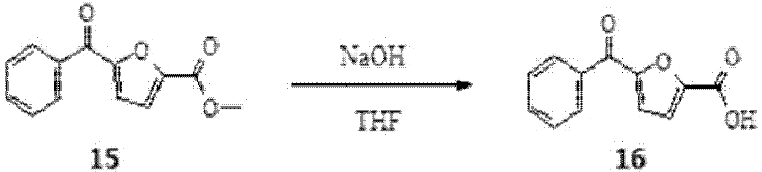

NaOH·H₂O (1.2 g, 30 mmol, 3 eq) was added to a solution obtained by dissolving compound 15 (2.9 g, 10 mmol, 1.0 eq) in THF (30 mL) cooled to 0°C followed by stirring at RT overnight. The reaction mixture was concentrated, and the residue was cooled, followed by acidification with 1N HCl of pH < 6 and extraction with EA (30 mL x 3). The collected organic layer was washed with water, dried over anhydrous Na₂SO₄, and concentrated. The residue was purified by chromatography charged with silica gel (PE/EA = 20/1) to give compound 16 as gray solid (2.2 g, 81.5%).

### Step 3:

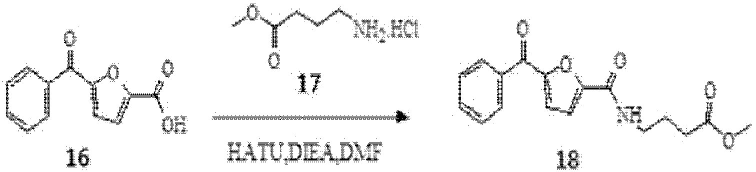

A solution obtained by dissolving DIEA (11.05 ml, 18.06 mmol, 3.0 eq), HATU (7.62 g, 6.02 mmol, 3.0 eq), compound 17 (3 g, 6.02 mmol, 1 eq), and compound 16 (4.4 g, 6.02 mmol, 1.0 eq) in DMF (250 mL) was stirred at RT overnight. The reaction mixture was cooled with water. The aqueous solution was extracted with EA (50 mL x 2) several times. The collected organic layer was washed with water, dried over anhydrous Na₂SO₄, and concentrated. The residue was purified by chromatography charged with silica gel (PE/EA = 2/1) to give compound 18 as yellow solid (3.2 g, 50%).

### Step 4:

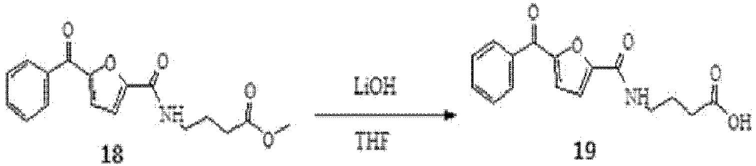

LiOH·H₂O (1.2 g, 27 mmol, 3 eq) was added to a solution obtained by dissolving compound 18 (3.2 g, 9.66 mmol, 1.0 eq) in THF (50 mL) cooled to 0°C followed by stirring at RT overnight. The reaction mixture was concentrated, and the residue was cooled, followed by acidification with 1N HCl of pH < 6, thereby obtaining an unpurified residue. The residue was purified by chromatography charged with silica gel (DCM/MeOH = 6/1) to give compound 19 as yellow solid (2.6 g, 85%).

### Step 5:

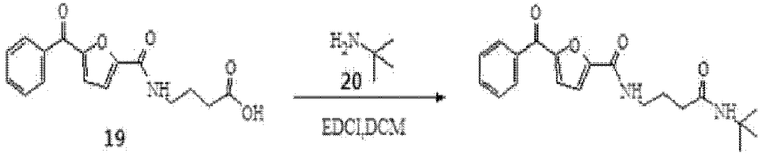

A solution obtained by dissolving EDCI (191 mg, 1.98 mmol, 3 eq), compound 20 (73 mg, 0.99 mmol, 1.5 eq), and compound 19 (200 mg, 0.66 mmol, 1.0 eq) in DCM (10 ml) was stirred at RT overnight. The reaction mixture was concentrated and the residue was diluted with water. The aqueous solution was extracted with EA (30 mL x 2) several times. The collected organic layer was washed with water, dried over anhydrous Na₂SO₄, and then concentrated. The residue was purified by preparative high performance liquid chromatography (prep-HPLC) to give a target compound as white solid (100 mg).

### Example 7: 5-Benzoyl-N-(3-(tert-butylamino)-3-oxopropyl)furan-2-carboxamide (FY-05-04-I-04)

A target compound was obtained by the same method as in example 6 except that, in step 4 in example 6, compound 21 below, instead of adding compound 17, was added, followed by reaction.

### <Compound 21>

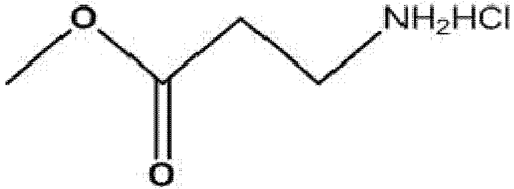

### Example 8: 5-Benzoyl-N-(4-(hydroxyamino)-4-oxobutyl)furan-2-carboxamide (FY-10-02-I-2)

### Step 1:

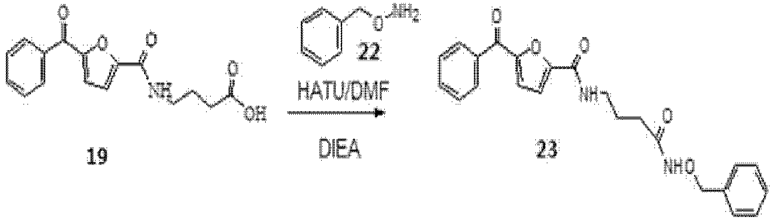

DIEA (255 mg, 1.98 mmol, 3 eq) was slowly added, under N₂, to a solution obtained by dissolving HATU (251 mg, 0.66 mmol, 1.0 eq), compound 22 (97.4 mg, 0.79 mmol, 1.2 eq), and compound 19 (200 mg, 0.66 mmol, 1.0 eq) in DMF (10 mL), followed by stirring at RT overnight. The reaction mixture was concentrated and the residue was diluted with water. The aqueous solution was extracted with EA (30 mL x 2) several times. The collected organic layer was washed with water, dried over anhydrous Na₂SO₄, and concentrated. The residue was purified by flash chromatography charged with silica gel (PE/EA = 1/2) to give compound 23 as colorless oil (400 mg, 100%).

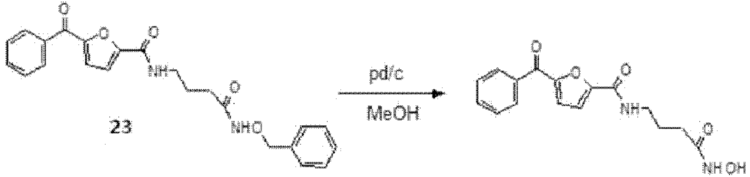

Pd/C was added to a solution obtained by compound 23 in MeOH, followed by stirring at 50°C under H₂ overnight. The reaction mixture was filtered and concentrated, and the unpurified residue was obtained. The residue was purified by preparative high performance liquid chromatography (HPLC) to give a white solid target compound (80 mg, 11%).

### Example 9: 5-(((2-Hydroxyethyl)(2-(2-methyl-1H-indol-3-yl)ethyl)amino)methyl)furan-2-carboxylic acid (FY-07-05-I-1A)

### Step 1:

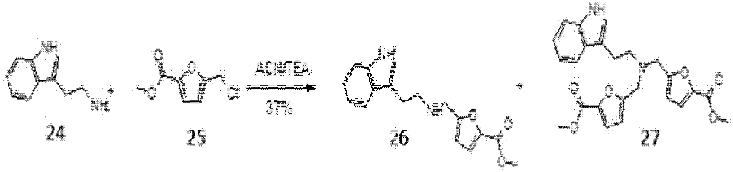

TEA (1.3 mL) was slowly added, under N₂, to a solution obtained by dissolving compound 24 (500 mg, 3.1 mmol, 1.0 eq) and compound 25 (650 mg, 3.7 mmol, 1.2 eq) in ACN (50 mL), followed by stirring at RT overnight. The reaction mixture was concentrated, and the brown oil reside was diluted with water. The aqueous solution was extracted with EA (100 mL x 2) several times. The collected organic layer was washed with water, dried over anhydrous Na₂SO₄, and concentrated. The residue was purified by flash chromatography charged with silica gel (PE/EA = 5/1) to give compound 27 100 mg, 7.4%) and compound 26 (350 mg, 37%) as colorless oil.

### Step 2:

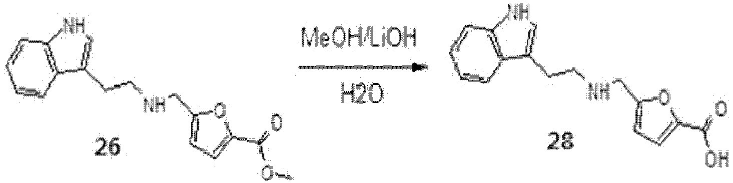

LiOH·H₂O (30 mg, 0.7 mmol, 1.5 eq) was added to a solution obtained by dissolving compound 26 (80 mg, 0.5 mmol, 1.0 eq) in MeOH/H₂O (2:1, 15 mL) cooled to 0°C followed by stirring at RT overnight. The reaction mixture was concentrated, and the residue was cooled, followed by acidification with 1N HCl of pH < 6 and concentration, thereby obtaining an unpurified residue. The residue was purified by preparative high performance liquid chromatography (prep-HPLC) to give compound 28 as white solid (140 mg, 97%).

### Step 3:

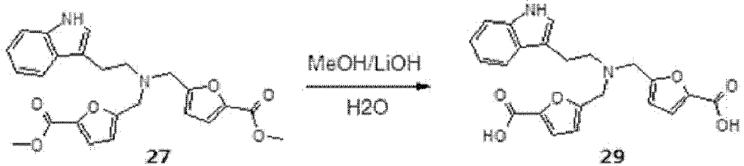

LiOH·H₂O (30 mg, 0.7 mmol, 3.0 eq) was added to a solution obtained by dissolving compound 27 (80 mg, 0.5 mmol, 1.0 eq) in MeOH/H₂O (2:1, 8 mL) cooled to 0°C followed by stirring at RT overnight. The reaction mixture was concentrated, and the residue was cooled, followed by acidification with 1N HCl of pH < 6 and concentration, thereby obtaining an unpurified residue. The residue was purified by preparative high performance liquid chromatography (prep-HPLC) to give compound 29 as white solid (57 mg, 76%).

### Step 4:

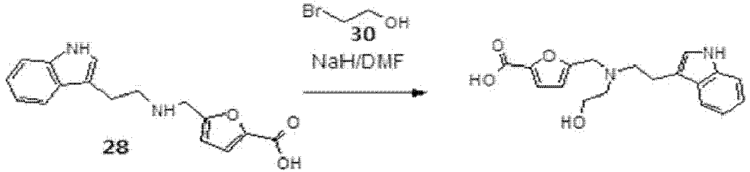

A solution obtained by dissolving compound 30 (65 mg, 0.5 mmol, 1.5 eq) in DMF was slowly added, under N₂, to a solution obtained by dissolving NaH (12 mg, 0.5 mmol, 1.5 eq) and compound 28 (100mg, 0.3 mmol, 1.0 eq) in DMF, followed by stirring at RT overnight. The reaction mixture was diluted with water and concentrated, thereby obtaining an unpurified residue. The residue was purified by preparative high performance liquid chromatography (HPLC) to give a white solid target compound (20 mg, 18.2%).

The target compound, 5-(((2-hydroxyethyl)(2-(2-methyl-1H-indol-3-yl)ethyl)amino)methyl)furan-2-carboxylic acid (FY-07-05-I-1A), was obtained by the same method as in example 9, except that compound 7 below, instead of compound 24 in step 1, was added, followed by reaction.

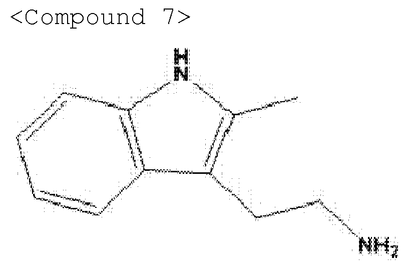

### Example 10: 1-(5-((3,4-Dihydroisoquinolin-2(1H)-yl)methyl)furan-2-yl)-2,2,2-trifluoroethanone (FY-07-07-I-1)

### Step 1:

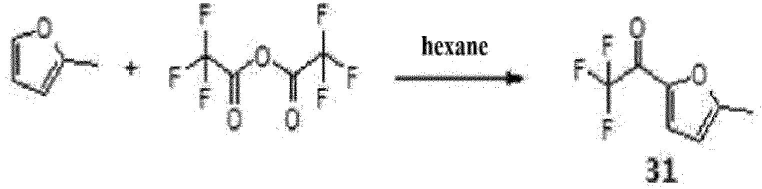

2-Methylfuran (11.0g, 134mmol, 1eq) was dissolved in hexane, followed by addition of 2,2,2-trifluoroacetyl-2,2,2-trifluoroacetate in, preferably, a drop manner, and the temperature was maintained at 0°C. After the addition, the reaction was conducted through stirring at the same temperature for 2 hours. The reaction mixture was cooled with ice water. The reaction mixture was extracted with EA, washed with saturated aqueous NaHCO₃, subsequently washed with brine and water, and dried over MgSO₄. Unpurified orange oil was used in the next step without further purification.

### Step 2:

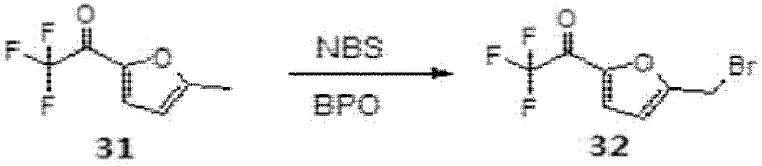

2,2,2-Trifluoro-1-(5-methyl(2-furyl))ethan-1-one (15 g, 84.2 mmol, 1 eq), NBS (15 g, 84.2 mmol, 1 eq), and BPO (1.1 g, 4.2 mmol, 0.05 eq) were dissolved in CCl₄, and the reaction mixture was stirred under reflux overnight. The reaction solution was filtered, and the filtrate was collected through condensation. The reacted unpurified product was separated by chromatography (EA/PE=1:50 - 1:20) to give orange oil (17 g, 78.8 %).

### Step 3:

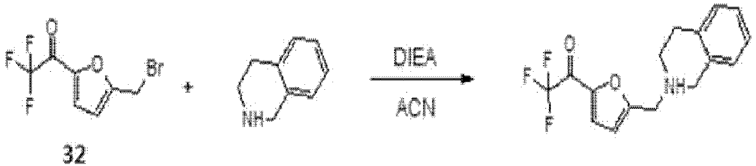

1,2,3,4-Tetrahydroisoquinoline (125 mg, 0.94 mmol, 0.95 eq) and DIEA (260 mg, 2.0 mmol, 2.0 eq) were dissolved in 3 mL of ACN. A solution obtained by dissolving 1-[5-(bromomethyl)(2-furyl)]-2,2,2-trifluoroethan-1-one (250mg, 0.97mmol, 1.0 eq) in 2 mL of ACN was slowly added. The reaction mixture was stirred for 1 hour. The reaction mixture was cooled with water, extracted with DCM, washed with brine and water, dried over MgSO₄, and concentrated. The reacted unpurified product was purified by preparative high performance liquid chromatography (HPLC) to give a target compound as white solid (132.8 mg, 86.8%).

### Example 11: 1-(5-(((2-(1H-indol-3-yl)ethyl)amino)methyl)furan-2-yl)-2,2,2-trifluoroethanone (FY-07-07- 1-2)

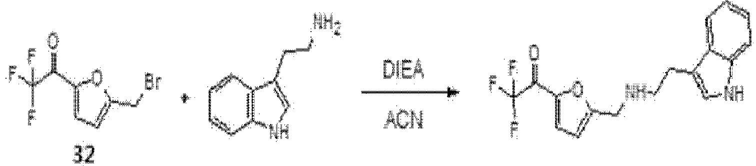

2-Indol-3-ylethyl amine (155 mg, 0.97 mmol, 1.0 eq) and DIEA (260 mg, 2.0 mmol, 2.0 eq) were dissolved in 3 mL of ACN. A solution obtained by dissolving 1-[5-(bromo ethyl) (2-furyl)]-2,2,2-trifluoroethan-1-one (250 mg, 0.97 mmol, 1.0 eq) in 2 mL of ACN was slowly added. The reaction mixture was stirred for 1 hour. The reaction mixture was cooled with water, extracted with DCM, washed with brine and water, dried over MgSO₄, and concentrated. The reacted unpurified liquid was purified by preparative high performance liquid chromatography (prep-HPLC) to give a target compound as brown solid (33.2 mg, 10.2%).

### Example 12: 2,2,2-Trifluoro-1-(5-(((2-(2-methyl-1H-indol-3-yl)ethyl)amino)methyl)furan-2-yl)ethanone (FY-07-09- I-1)

A target compound was obtained by the same method as in example 11 except that in the preparation step of example 11, compound 7 below, instead of 2-indol-3-ylethyl amine, was added.

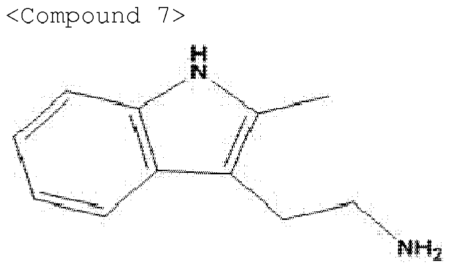

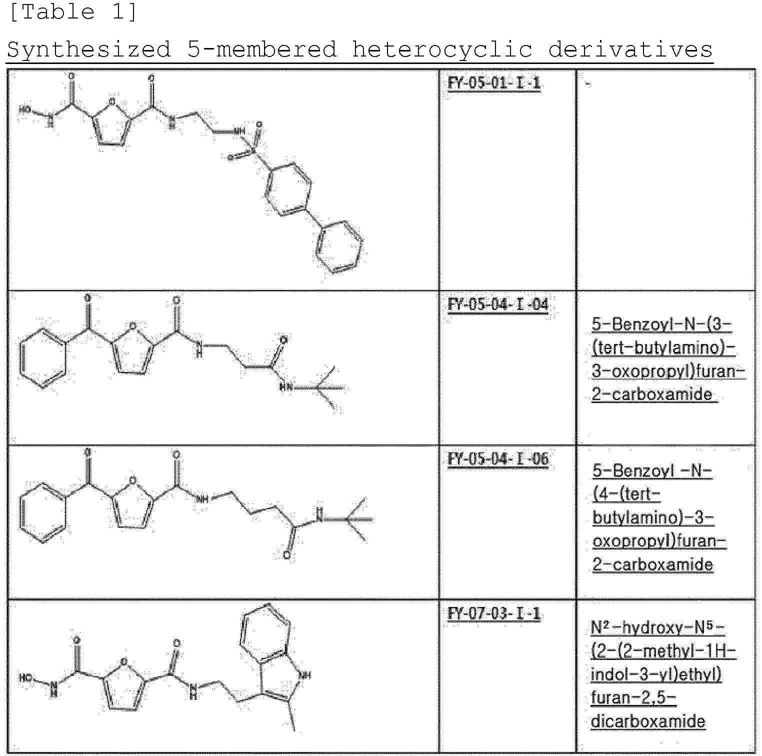

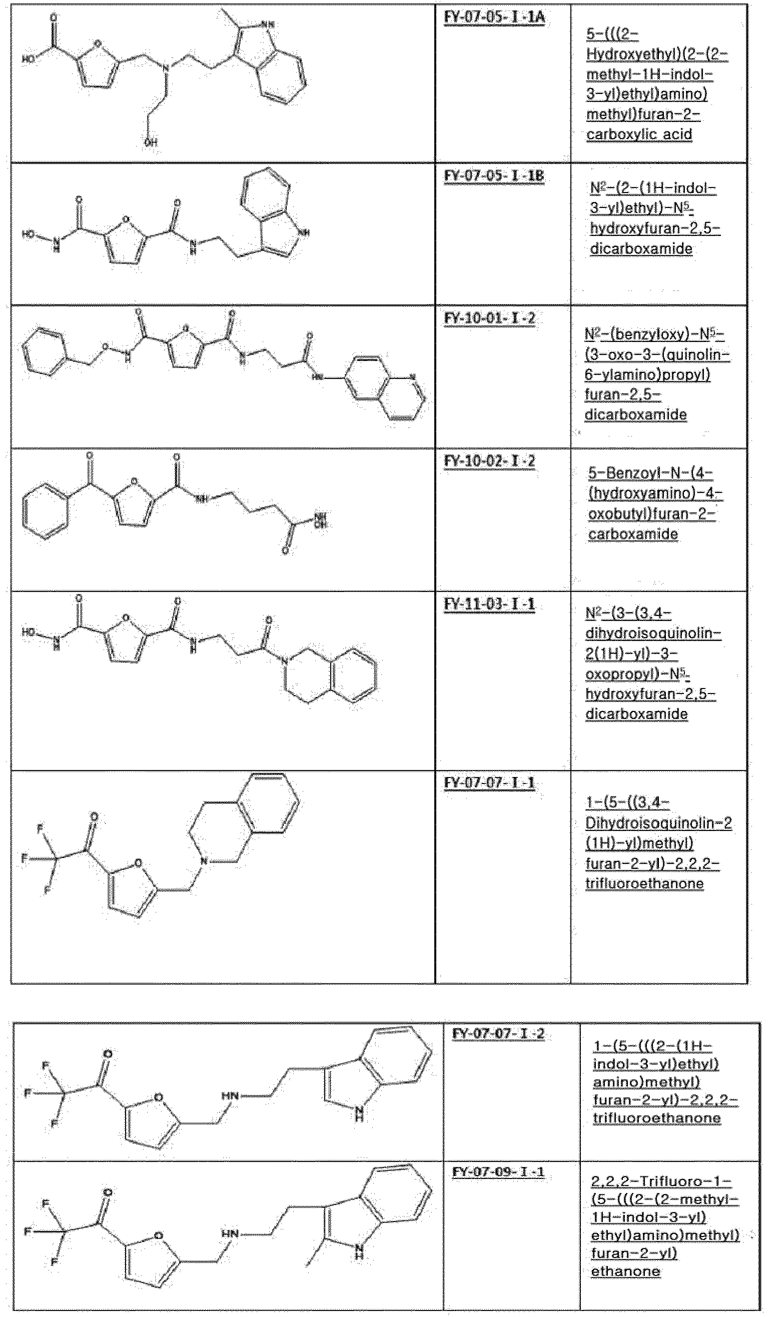

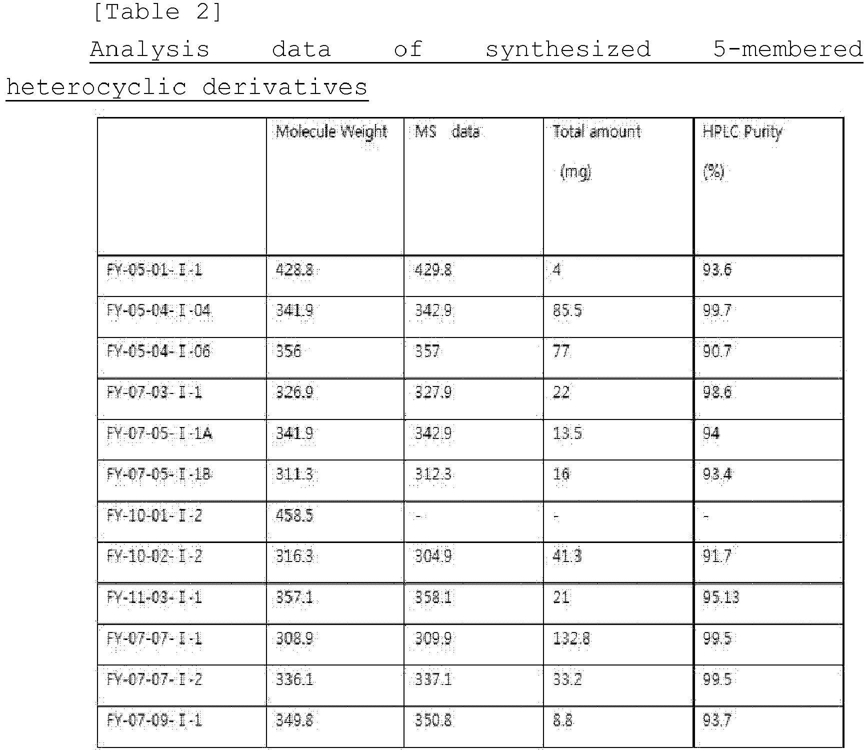

### Test Example 1: Writhing inhibition test of synthesized 5-membered heterocyclic derivatives

4-Week-old ICR mice (body weight: about 25 g) were orally, intraspinally, and intraventricularly administered with the 5-membered heterocyclic derivatives of the present invention. Then, the mice were abdominally administered with 0.25 mℓ of 1% acetic acid, and then subjected to a writhing response test for 30 minutes. Here, mice administered without the 5-membered heterocyclic derivatives of the present invention, as a control, were tested in the same manner.

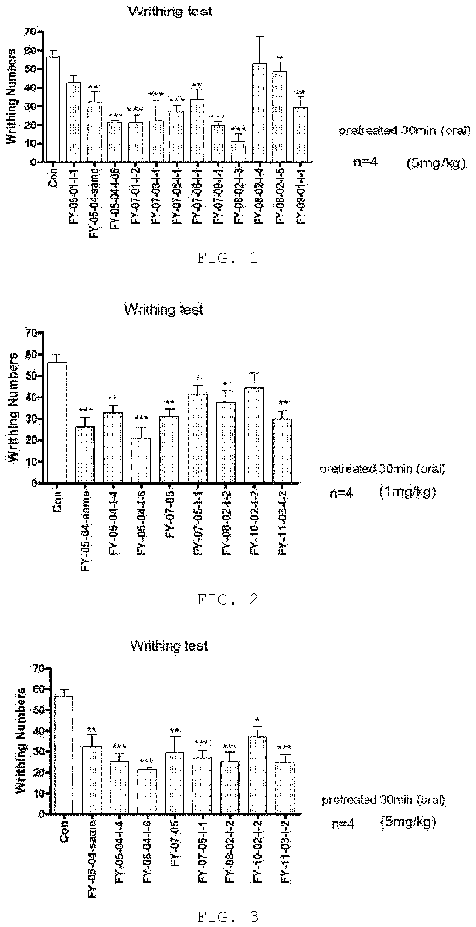

FIGS. 1 to 5 show test results (writhing test) in the oral administration of the 5-membered heterocyclic derivatives of the present invention. The mice were orally administered with the 5-membered heterocyclic derivatives of the present invention at a concentration of 1 mg/kg or 5 mg/kg, and after 30 minutes, subjected to a writhing test by abdominal administration of 1% acetic acid. The results confirmed a significant analgesic effect.

### Test Example 2: Formalin test of synthesized 5-membered cyclic derivatives

The 4-week-old ICR mice (body weight: about 25 g) were orally, intraspinally, and intraventricularly administered with the 5-membered heterocyclic derivatives of the present invention. After 30 minutes, a 5% aqueous formalin solution was administered to a hind paw sole of each mouse, and then the duration of nociceptive behavior, such as licking or shaking the sole, was measured for the first phase of 5 minutes and the second phase of 20-40 minutes. Here, mice administered without the 5-membered heterocyclic derivatives of the present invention, as a control, were tested in the same manner.

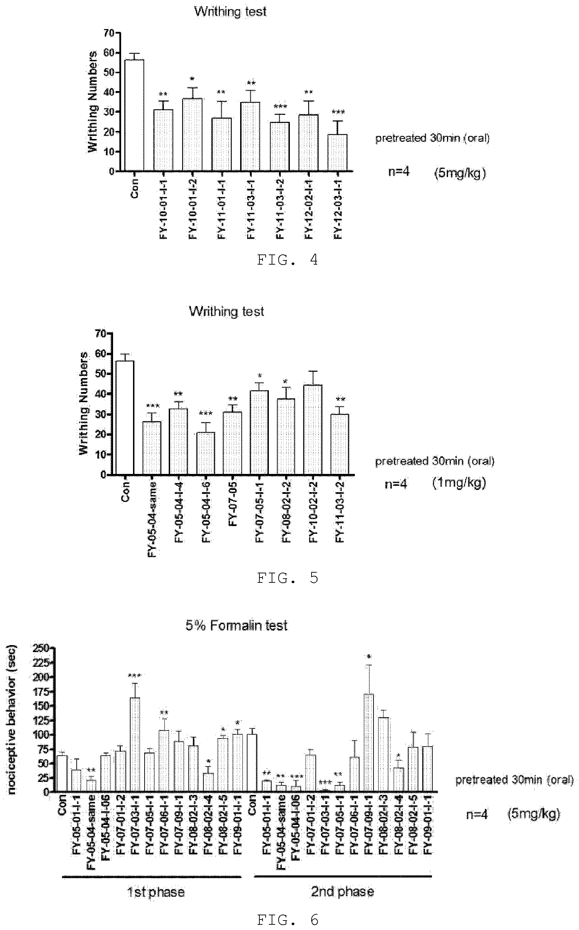

FIGS. 6 to 8 show test results (formalin test) in the oral administration of the 5-membered heterocyclic derivatives of the present invention. The mice were orally administered with the 5-membered heterocyclic derivatives of the present invention at a concentration of 5 mg/kg, and after 30 minutes, subjected to a test by administration of 5% formalin into the left paw sole of each mouse. The results confirmed an analgesic effect for both the first and second phases.

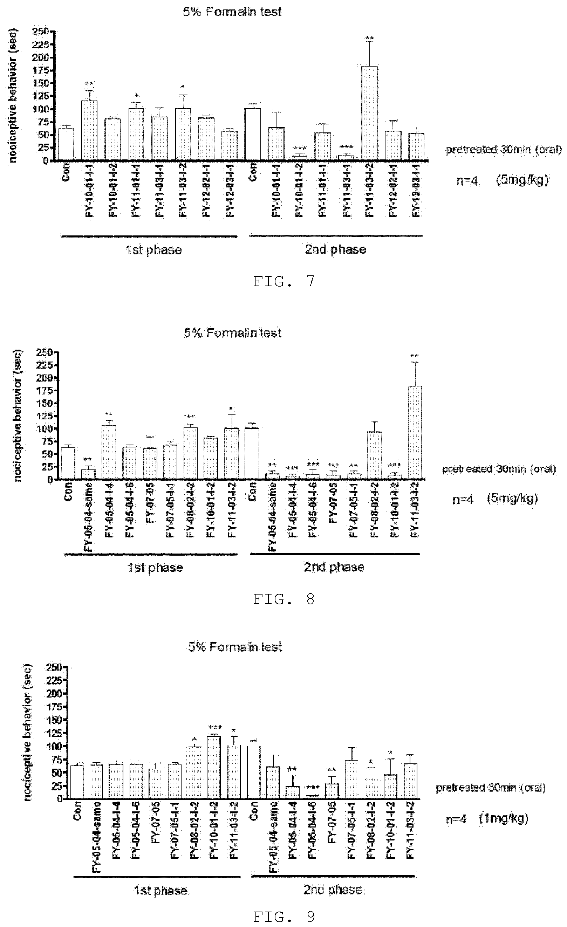

FIG. 9 shows that the mice were orally administered with the 5-membered heterocyclic derivatives of the present invention at a concentration of 1 mg/kg, and after 30 minutes, subjected to a test by administration of 5% formalin into the left paw sole of each mouse, and the results confirmed an analgesic effect for both the first and second phase.

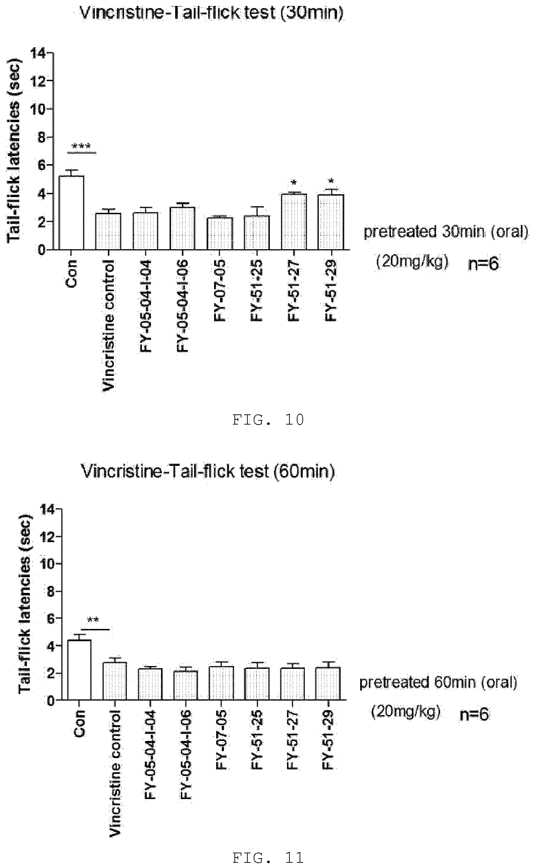

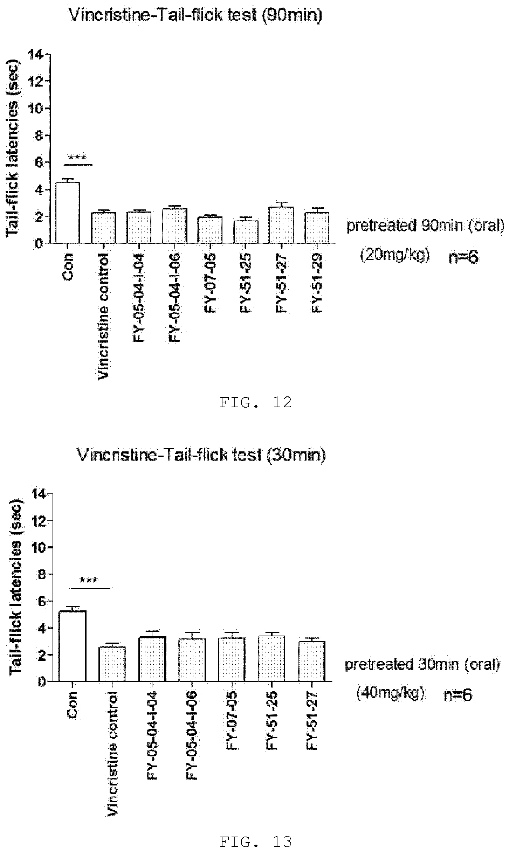

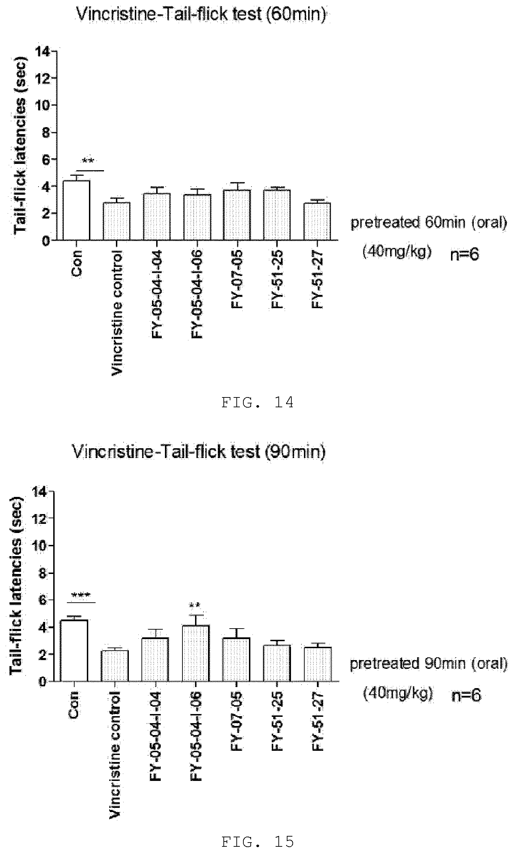

FIGS. 10 to 15 depict graphs showing analgesic effects, over time, of the 5-membered heterocyclic derivatives of the present invention in the neuropathic pain models induced by vincristine. It could be confirmed from the above graphs that the 5-membered heterocyclic derivatives of the present invention had an analgesic effect on neuropathic pain in a dose-dependent manner, and showed a significant analgesic effect from 30 minutes and an excellent analgesic effect at 60 and 90 minutes.

### Test Example 3: Evaluation of basic properties of 5-benzoyl-N-(4-(tert-butylamino)-3-oxopropyl)furan-2-carboxamide(FY-05-04-I-06)

### (1) pKa

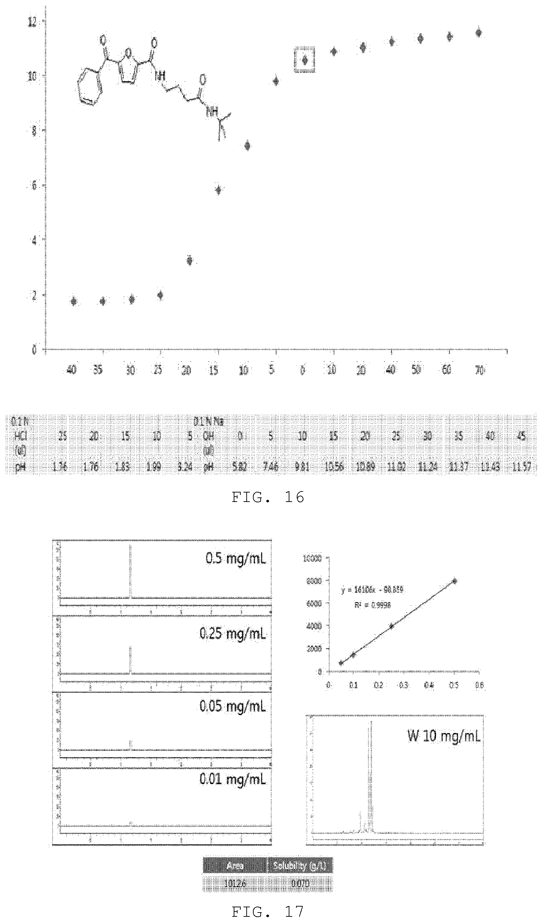

In the pKa test, 2-10 mg of a sample was dissolved in 1 mL of HPLC grade MeOH, and then the pH change was measured while 1-10 uL of 0.1 N NaOH or 0.1 HCL was added. As a result, the pKa value was measured as 12.1 as shown in FIG. 16.

### (2) Solubility

As for the measurement of solubility, 10 mg of a target compound sample was diluted to concentrations of 1.0, 0.5, 0.25, 0.05, and 0.01 mg/mL by being dissolved in 1 mL of HPLC grade MeOH, followed by content measurement using HPLC, thereby obtaining a standard curve. Thereafter, 10 mg of each compound was dissolved in 1 mL of distilled water, sonicated for 1 hour, and centrifuged (13,000 rpm x 20 min), and then 100 uL of the supernatant was mixed with 900 uL of distilled water to make a final concentration of 1 mg/mL, followed by the measurement of solubility using HPLC.

As a result, as shown in FIG. 17, the solubility of 5-benzoyl -N-(4-(tert-butyl amino)-3-oxopropyl)furan-2-carboxamide (FY-05-04-I-06) was measured as 0.07 g/L.

### (3) Measurement of LogP and permeability

Log P and permeability were measured as shown in Table 3.

**[Table 3]**

| Compound | FY-504 | comment |
|---|---|---|
| Log P | **1.45 (XH+: 0.89) (neutral X: 1.45)** | Lipophilicity |
| Permeability | **- 4.35 ± 0.0321 (grade : medium)** | high : -4.07 < |
| | | medium : -4.07^{∼}-4.87 |
| | | low : -4.87 > |

### (4) In-sillico ADME (pre ADME)

Only the absorption and distribution of a drug were investigated using PRE-ADME (http://preadmet.bmdrc.org/index.php), which is a virtual screening program developed in Korea. Results were shown in Table 4.

**[Table 4]**

| Drug absorption and distribution | | | |
|---|---|---|---|
| Pre-AD | | Value | |
| | | Ref | FY-504(FY-05-04-I-06) |
| **Absorption** | Human intestinal absorption (HIA, %) | ≥ 50 | 93.6 |
| | in vitro Caco-2 cell permability (nm/sec) | ≥ 4 | 11.4 |
| | in vitro MDCK cell permability (nm/sec) | ≥ 25 | 77.9 |
| Distribution | in vitro skin permeability (logKp, cm /hour) | - | -2.53 |
| | in vitro-plasma protein binding (%) | ≤ 90 | 62.7 |
| | in vitro blood-brain barrier penetration (C. brain/C. blood) | ≤ 0.1 | 0.18 |

### Test Example 4: Measurement of effect of 5-benzoyl-N-(4-(tert-butylamino)-3-oxopropyl)furan-2-carboxamide(FY-05-04- I-06) on voltage-gated Na channel

The α subunits of the Na channel are largely classified into TTX-sensitive channels and TTX-resistant Na channels. In this experiment, small-sized neurons (diameter < 30 *µ*m) known as pain neurons were selected and recorded. TTX-sensitive Na channels are divided into NaV 1.1, 1.2, 1.3, 1.4, 1.6, and 1.7, and TTX-resistant Na channels are divided into NaV 1.5, 1.8, and 1.9. According to many papers, especially NaV 1.8 is known to be closely associated with neuropathic pain.

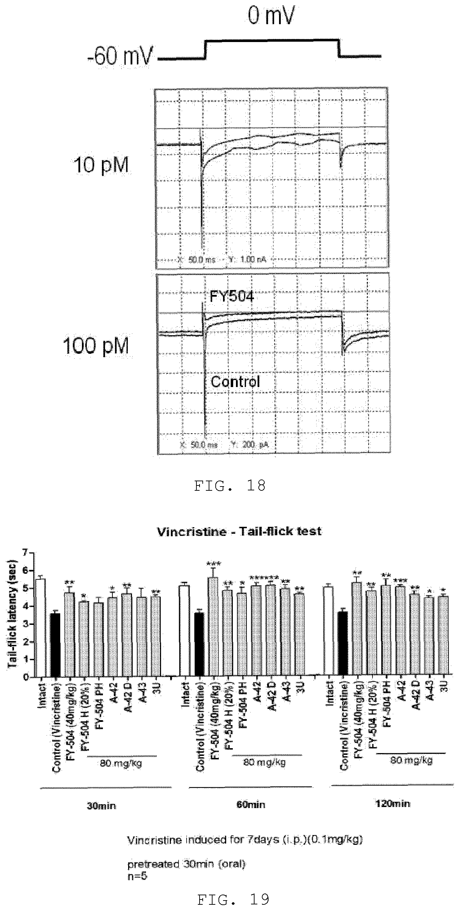

In order to evaluate the blocking effect on the TTX-resistant Na channels, the blocking effects on the Na channel for various concentrations were investigated. As a result, it was confirmed that Na current was completely blocked at a low concentration, even 100 pM, and about 50% blocking of Na current was observed at 10 pM (FIG. 18). The efficacy evaluation of 5-benzoyl -N-(4-(tert-butylamino)-3-oxopropyl)furan-2-carboxamide(FY-05-04-1-06) on the TTX-sensitive Na channels was carried out, and the results confirmed that 5-benzoyl -N-(4-(tert-butylamino)-3-oxopropyl) furan-2-carboxamide (FY-05-04-1-06) did not have an effect on TTX-sensitive Na current. The inhibition of TTX-resistant Na channels, which are important in the signaling of neuropathic pain was resultantly confirmed.

Resultantly, the structure of 5-benzoyl-N-(4-(tert-butylamino)-3-oxopropyl)furan-2-carboxamide(FY-05-04-I-06) compound is a material group that is completely different from the currently developed TTX-r Nav structures, and thus a novel compound. The IC50 value for the currently confirmed TTX-r Nav is less than 10 pM, which is 1000 times better than the Nav Blockers that has currently been developed by competitors.

### Test Example 5: Measurement of effects of 5-benzoyl-N-(4-(tert-butylamino)-3-oxopropyl)furan-2-carboxamide (FY-05-04-I-06) in vincristine neuropathy models

### 1) Vincristine neuropathy model

Neuropathy was induced by abdominal administration of vincristine at 0.1 mg/kg once a day for seven days. The symptoms of neuropathic pain began to appear from the 4th day after administration, and significant pain was shown on the 7th day. The degree of neuropathic pain was measured through the Tail-flick test and von-Frey test (Junzo et al., 2005).

### 2) Tail-flick test

The analgesic effect was measured by placing a mouse on the machine and measuring an avoidance response of the tail, which is caused by application of heat. The intensity was adjusted so that the response time was 3-4 seconds, and the cut off time was set to about 10 seconds (to prevent tissue damage of the mouse tail).

In neuropathic animal models in which neuropathy was induced by abdominal administration of vincristine at 0.1 mg/kg once a day for 7 days, each compound was orally administered at a concentration of 40 or 80 mg/kg, and after 30, 60, and 120 minutes therefrom, the degree of analgesia was measured by a tail-flick test. The results are shown in FIG. 19. It was confirmed that FY-504 showed an analgesic effect of 63% for 30 minute and 127% for 60 minutes; FY-504H (20%) showed an analgesic effect of 36% for 30 minutes, 82% for 60 minutes, and 80% for 120 minutes; A-42 showed an analgesic effect of 50% for 30 minutes, 100% for 60 minutes, and 100% for 120 minutes; A-42D showed an analgesic effect of 57% for 30 minutes, 100% for 60 minutes, and 60% for 120 minutes; A-43 showed an analgesic effect of 50% for 30 minutes, and 82% for 60 minutes, and 50% for 120 minutes; and 3U showed an analgesic effect of 50% for 30 minutes, 64% for 60 minutes, and 50% for 120 minutes.

### 3) Von Frey filament test

An experimental rat was placed on a metal mesh (mesh spacing, 0.5 cm x 0.5 cm), placed in a transparent acrylic box (8 x 8 x 24 cm), and then adapted for 15 minutes to avoid stress induced by environmental changes. When the rat became still after adaptation, the weight at which the paw withdraw response appeared was recorded while a stimulator with von Frey filament (Semmes-Weinstein monofilaments, Stoelting, Wood Dale, IL, USA) in a pressure range of 0.356-18 g was used to apply the filament to a predetermined (left) paw sole through the iron mesh. When the filament was in vertical contact with the left paw sole for 5-6 seconds and then separated from the paw sole, the rat was considered to show a positive response if the rat mouse immediately flinched, showed a rapid withdraw response, or licked the paw (Kim et al., 2004).

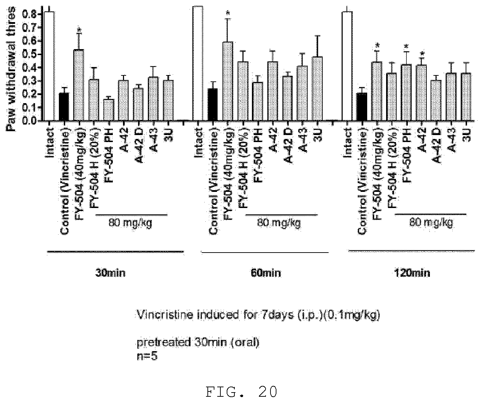

In neuropathic animal models in which neuropathy was induced by abdominal administration of vincristine at 0.1 mg/kg once a day for 7 days, each compound was orally administered at a concentration of 40 or 80 mg/kg, and after 30, 60, and 120 minutes therefrom, the degree of analgesia was measured by a von-Frey test. The results are shown in FIG. 20. It was confirmed that FY-504 showed an analgesic effect of 54% for 30 minute and 56% for 60 minutes; FY-504H (20%) showed an analgesic effect of 19% for 30 minutes, 34% for 60 minutes, and 27% for 120 minutes; A-42 showed an analgesic effect of 16% for 30 minutes, 34% for 60 minutes, and 35% for 120 minutes; A-42D showed an analgesic effect of 8% for 30 minutes, 15% for 60 minutes, and 16% for 120 minutes; A-43 showed an analgesic effect of 23% for 30 minutes, 26% for 60 minutes, and 23% for 120 minutes; and 3U showed an analgesic effect of 16% for 30 minutes, 37% for 60 minutes, and 23% for 120 minutes.

## Claims

1. A compound of chemical formula 1 below or a pharmaceutically acceptable salt thereof: 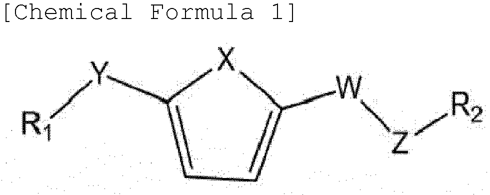 wherein,
X is O, S, or NH;
Y is selected from the group consisting of -C(O)-, -C(S)-, -C(NH)-, and unsubstituted or substituted linear or branched C₁-C₆ alkylene;
R₁ and R₂ each are independently selected from the group consisting of H, -NR'R", -C(O)NR'R", -OR₃, halogen, unsubstituted or substituted linear or branched C₁-C₁₀ alkyl, unsubstituted or substituted C₂-C₁₀ alkenyl, unsubstituted or substituted C₃-C₂₀ cycloalkyl, unsubstituted or substituted C₆-C₂₀ aryl, unsubstituted or substituted C₃-C₂₀ heterocyclyl, unsubstituted or substituted C₅-C₁₂ heteroaryl, and C₁-C₆ alkyl C₅-C₁₂ heteroaryl;
R₃ is H or linear or branched C₁-C₆ alkyl;
W is selected from the group consisting of a bond, -C(O)-NH-, -NH-C(O)-, and -C₁-C₆ alkylene-NR'R";
Z is a bond or linear or branched C₁-C₁₀ alkylene; and
R' and R" each are independently selected from the group consisting of H, unsubstituted or substituted linear or branched C₁-C₆ alkyl; C₃-C₁₂ cycloalkyl; unsubstituted or substituted 5- to 12-membered heterocyclyl; C₂-C₆ alkenyl; C₆-C₁₂ aryl; C₁-C₆ alkyl C₆-C₁₂ aryl; unsubstituted or substituted 5- to 12-membered heteroaryl; C₁-C₆ alkyl C₅-C₁₂ heteroaryl; C₁-C₆ alkyl sulfonyl; C₆-C₁₂ aryl sulfonyl; C₆-C₂₀ aryl sulfonamide; mercapto; hydroxy (C₁-C₁₀) alkyl; -OH; C₁-C₆ alkoxy; C₁-C₆ alkoxy C₁-C₁₀ alkyl; hydroxy C₁-C₁₀ alkyl; C₁-C₆ haloalkoxy; halogen; -COOH; -CHO; -CN; -C₁-C₆ alkyl carbonyl; -C₁-C₆ alkyl oxy carbonyl; and mono- or di-C₁-C₆ alkyl carbamoyl,
wherein R' and R", together with N, may form a saturated or unsaturated 3- to 6-membered ring, and the ring may be further substituted with C₆-C₂₀ aryl or C₅-C₁₂ heteroaryl or may form a fused ring together therewith, and
wherein the substituted alkyl, substituted alkenyl, substituted cycloalkyl, substituted aryl, substituted heterocyclyl, and substituted heteroaryl mean that the alkyl, alkenyl, cycloalkyl, aryl, heterocyclyl, and heteroaryl each are independently substituted with at least one substituent selected from the group consisting of C₁-C₁₀ alkyl; C₃-C₁₂ cycloalkyl; C₂-C₁₀ alkenyl; C₆-C₂₀ aryl; (C₁-C₆)alkyl(C₆-C₁₂)aryl; C₅-C₁₂ heteroaryl; C₁-C₆ alkyl sulfonyl; C₆-C₁₂ aryl sulfonyl; C₆-C₂₀ aryl sulfonamide; C₁-C₆ alkyl thio; mercapto; hydroxy(C₁-C₁₀)alkyl; -OH; C₁-C₆ alkoxy; C₆-C₂₀ aryloxy; (C₁-C₆)alkoxy(C₁-C₁₀)alkyl; C₁-C₆ haloalkoxy; -NO₂; halogen; - COOH; -CHO; -CN; -C₁-C₆ alkyl carbonyl; -C₁-C₆ alkyl oxy carbonyl; -CONR'R"; -NR'R"; N-hydroxyl carbamoyl; and mono- or di-C₁-C₁₀ alkyl carbamoyl.

2. The compound or pharmaceutically acceptable salt thereof of claim 1, wherein X is O.

3. The compound or pharmaceutically acceptable salt thereof of claim 1, wherein Y is -C(O)-.

4. The compound or pharmaceutically acceptable salt thereof of claim 1, wherein X is O;
Y is -C(O)-;
R₁ is unsubstituted or substituted C₆-C₂₀ aryl, unsubstituted or substituted C₅-C₁₂ heteroaryl, or - NR'R", in which R' and R" are as defined in claim 1.

5. The compound or pharmaceutically acceptable salt thereof of any one of claims 1 to 4, wherein W is - C(O)-NH- or linear or branched C₁-C₁₀ alkylene;
Z is a bond or linear or branched C₁-C₁₀ alkylene; and
R₂ is -NR'R", -C(O)NR'R", -C₁-C₆ alkyl oxy carbonyl, -COOH, C₆-C₂₀ aryl, unsubstituted or substituted C₃-C₂₀ heterocyclyl, or unsubstituted or substituted C₅-C₁₂ heteroaryl, in which R' and R" are as defined in claim 1.

6. The compound or pharmaceutically acceptable salt thereof of claim 5, wherein W is -C(O)-NH-;
Z is linear or branched C₁-C₁₀ alkylene;
R₂ is -NR'R", -C(O)NR'R", -C₁-C₆ alkyl oxy carbonyl, -COOH, C₆-C₂₀ aryl, unsubstituted or substituted C₃-C₂₀ heterocyclyl, or unsubstituted or substituted C₅-C₁₂ heteroaryl; and
R' and R" each are H or linear or branched C₁-C₆ alkyl.

7. A compound of chemical formula 1 or a pharmaceutically acceptable salt thereof, wherein the compound of chemical formula 1 of claim 1 is selected from the following compounds:
5-benzoyl-N-(3-(tert-butylamino)-3-oxopropyl)furan-2-carboxamide;
5-benzoyl-N-(4-(tert-butylamino)-3-oxopropyl)furan-2-carboxamide;
N²-hydroxy-N⁵-(2-(2-methyl-1H-indol-3-yl)ethyl)furan-2,5-dicarboxamide;
5-(((2-hydroxyethyl)(2-(2-methyl-1H-indol-3-yl)ethyl)amino)methyl)furan-2-carboxylic acid;
N²-(2-(1H-indol-3-yl)ethyl)-N⁵-hydroxyfuran-2,5-dicarboxamide;
N²-(benzyloxy)-N⁵-(3-oxo-3-(quinolin-6-ylamino)propyl)furan-2,5-dicarboxamide;
5-benzoyl-N-(4-(hydroxyamino)-4-oxobutyl)furan-2-carboxamide;
N²-(3-(3,4-dihydroisoquinolin-2(1H)-yl)-3-oxopropyl)-N⁵-hydroxyfuran-2,5-dicarboxamide;
1-(5-((3,4-dihydroisoquinolin-2(1H)-yl)methyl)furan-2-yl)-2,2,2-trifluoroethanone;
1-(5-(((2-(1H-indol-3-yl)ethyl)amino)methyl)furan-2-yl)-2,2,2-trifluoroethanone;
and
2,2,2-trifluoro-1-(5-(((2-(2-methyl-1H-indol-3-yl)ethyl)amino)methyl)furan-2-yl)ethanone.

8. A pharmaceutical composition for treating or relieving neuropathic pain, acute pain, chronic pain, abdominal pain, toothache, menstrual pain, backache, frozen shoulder pain, herpes zoster, trigeminal neuralgia, cancer pain, diabetic neuropathy, post-operative pain, migraine, or joint pain, the pharmaceutical composition containing the compound of chemical formula 1 or pharmaceutically acceptable salt thereof of claim 1.
